# EUROPEAN PATENT APPLICATION

(11) **EP 3 747 955 A1**
(43) Date of publication of application: **09.12.2020**
(21) Application number: 19746821.8
(22) Date of filing: 31.01.2019
(51) Int. Cl.: C09B 11/24, C09B 67/20, G01N 21/78, G01N 31/00, G01N 33/52

(54) **ACETYLPOLYAMINE DETECTION AGENT**

(30) Priority: 31.01.2018 JP 2018014792
(71) Applicant: DAIKIN INDUSTRIES, LTD., Osaka 530-8323 (JP)
(72) Inventor: HAMADA, Tomohito, Osaka-shi, Osaka 530-8323 (JP); KISHIKAWA, Yosuke, Osaka-shi, Osaka 530-8323 (JP); SUKEGAWA, Masamichi, Osaka-shi, Osaka 530-8323 (JP)
(74) Representative: Hoffmann Eitle
(86) International application number: PCT/JP2019/003329
(87) International publication number: WO 2019/151381

(57) **Abstract**

A main object of the present invention is to provide a novel acetylpolyamine detection agent that is easy to produce and use.

This object is achieved by an acetylpolyamine detection agent comprising compound (A) having one or more polyether rings, wherein the polyether rings have one or more hydroxyl groups as substituents.

## Description

### Technical Field

The present invention relates to an acetylpolyamine detection agent.

### Background Art

It has been reported that the amounts of N1,N8-diacetylspermidine and N1,N12-diacetylspermine, which are kinds of acetylpolyamine, are useful in distinguishing patients with malignancies from healthy persons (NPL 1).

Further, a sensitive and accurate enzyme-linked immunosorbent assay (ELISA) system for N1,N12-diacetylspermine has been proposed (NPL 2).

However, the technique described in NPL 2 is disadvantageous in terms of ease of production and use because antibodies are used.

A main object of the present invention is to provide a novel acetylpolyamine detection agent that is easy to produce and use.

### Citation List

### Non-patent Literature

NPL 1: Sugimoto M, et al., J. Cancer Res. Clin. Oncol., 121, 317-319 (1995)
NPL 2: Hiramatsu K, Miura H, Kamei S, Iwasaki K, and Kawakita M: Development of a sensitive and accurate enzyme-linked immunosorbent assay (ELISA) system that can replace HPLC analysis for the determination of N1,N12-diacetylspermine in human urine, J. Biochem., 124: 231-236, 1998

### Summary of Invention

### Technical Problem

The technique described in PTL 1 is disadvantageous in terms of ease of production and use because antibodies are used.

A main object of the present invention is to provide a novel acetylpolyamine detection agent that is easy to produce and use.

### Solution to Problem

As a result of intensive studies, the present inventors found that the above object can be achieved by an acetylpolyamine detection agent comprising compound (A) having one or more polyether rings, wherein the polyether rings have one or more hydroxyl groups as substituents. The present invention has thus been completed.

The present invention includes the following aspects.
Item 1.
   An acetylpolyamine detection agent comprising compound (A) having one or more polyether rings, wherein the polyether rings have one or more hydroxyl groups as substituents.
Item 2.
   The acetylpolyamine detection agent according to Item 1, wherein compound (A) is a compound represented by formula (A1) : 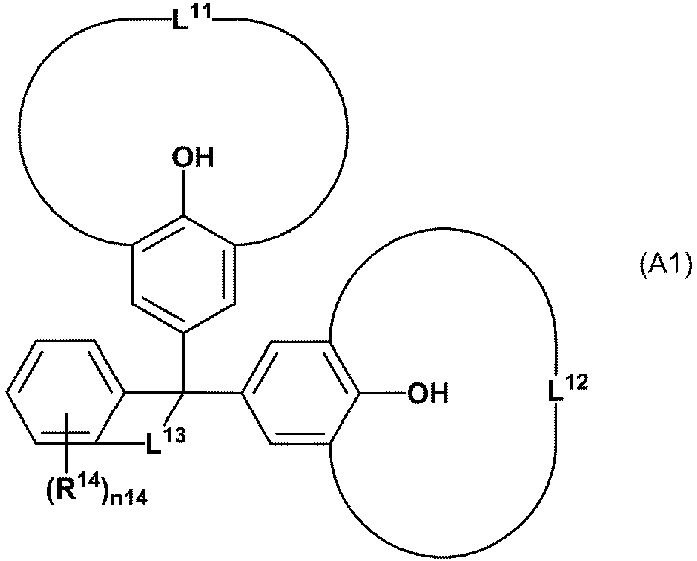 wherein
   L¹¹ is a divalent polyether chain,
   L¹² is a divalent polyether chain,
   L¹³ is a divalent group that can be cleaved at the end or inside, n¹⁴ is a number of 0 to 4, and
   R¹⁴ is independently at each occurrence a substituent, or two of R¹⁴ may form a substituted or unsubstituted ring together with carbon atoms to which they are bonded.
Item 3.
   The detection agent according to Item 2, wherein L¹¹ is:
   (1) -CH₂-(OCH₂CH₂)ₙ₁₁₁-OCH₂-, wherein n111 is a number of 1 to 10, or
   (2) -(Tz)ₙ₁₁₂-, wherein Tz is a glucose residue, and n112 is a number of 1 to 10.
Item 4.
   The detection agent according to Item 2 or 3, wherein L¹² is:
   (1) -CH₂-(OCH₂CH₂)ₙ₁₂₁-OCH₂-, wherein n121 is a number of 1 to 10, or
   (2) -(Tz)ₙ₁₂₂-, wherein Tz is a glucose residue, and n122 is a number of 1 to 10.
Item 5.
   The detection agent according to any one of Items 2 to 4, wherein L¹³ is -CO₂-, -SO₃-, or -CONH-.
Item 6.
   The detection agent according to any one of Items 2 to 5, wherein R¹⁴ is independently at each occurrence -F, -Cl, -Br, - CF₃, -NMe₂, or -OMe.
Item 7.
   The detection agent according to any one of Items 1 to 6, for use in detection of acetylpolyamine in a water-containing sample.
Item 8.
   The detection agent according to any one of Items 1 to 7, wherein the water-containing sample is a biological sample.
Item 9.
   The detection agent according to any one of Items 1 to 8, for use in diagnosis of a disease associated with a change in the acetylpolyamine concentration of the body fluid of an affected patient.
Item 10.
   The detection agent according to any one of Items 1 to 9, for use in tumor diagnosis.
Item 11.
   The detection agent according to any one of Items 1 to 10, wherein the acetylpolyamine is diacetylpolyamine.
Item 12.
   The detection agent according to any one of Items 1 to 11, wherein the acetylpolyamine is N¹,N¹²-diacetylspermine.
Item 13.
   A detection kit comprising the detection agent according to any one of Items 1 to 12.
Item 14.
   A method for detecting acetylpolyamine, comprising bringing the detection agent according to any one of Items 1 to 12 into contact with a sample having a possibility of containing acetylpolyamine.
Item 15.
   A composition comprising:
   a compound represented by formula (A1) or a ring-opened form thereof: 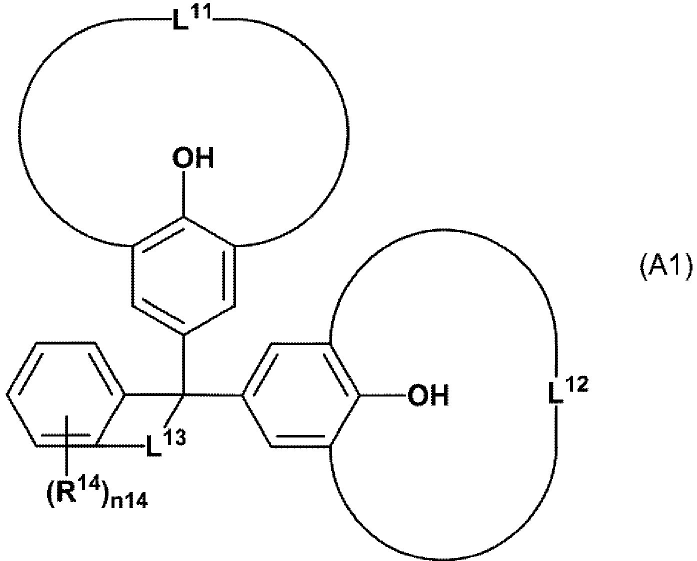 wherein
   L¹¹ is a divalent polyether chain,
   L¹² is a divalent polyether chain,
   L¹³ is a divalent group that can be cleaved at the end or inside, n¹⁴ is a number of 0 to 4, and
   R¹⁴ is independently at each occurrence a substituent, or two of R¹⁴ may form a substituted or unsubstituted ring together with carbon atoms to which they are bonded; and
      acetylpolyamine.
Item 16.
   A complex comprising:
   a compound represented by formula (A1) or a ring-opened form thereof: 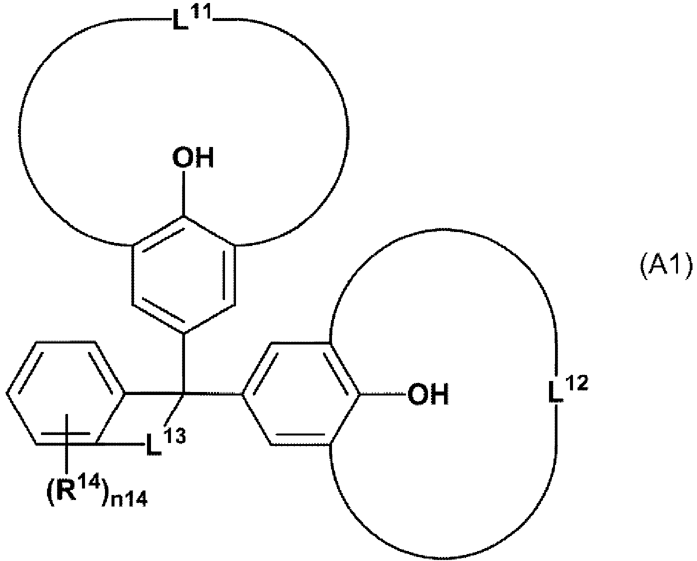 wherein
   L¹¹ is a divalent polyether chain,
   L¹² is a divalent polyether chain,
   L¹³ is a divalent group that can be cleaved at the end or inside, n¹⁴ is a number of 0 to 4, and
   R¹⁴ is independently at each occurrence a substituent, or two of R¹⁴ may form a substituted or unsubstituted ring together with carbon atoms to which they are bonded; and
      acetylpolyamine;
   wherein the acetylpolyamine is held in at least one of the ring containing L¹¹ and the ring having L¹² of the compound represented by formula (A1) or the ring-opened form thereof.

### Advantageous Effects of Invention

According to the present disclosure, provided are a novel acetylpolyamine detection agent and the like that are easy to produce and use.

### Description of Embodiments

### 1. Terms

Unless otherwise specified, the symbols and abbreviations in the present specification can be understood in the sense commonly used in the technical field to which the present invention pertains, according to the context of the present specification.

In the present specification, the terms "contain" and "comprise" are used with the intention to include the terms "consist essentially of" and "consist of."

Unless otherwise specified, the steps, treatments, or operations described in the present specification can be performed at room temperature.

In the present specification, room temperature means a temperature in the range of 10 to 40°C.

In the present specification, the notation "Cn-Cm" (where n and m are numbers) indicates that the number of carbon atoms is n or more and m or less, as is commonly understood by a person skilled in the art.

In the present specification, the phrase "may have a substituent" can mean substitution or unsubstitution.

In the present specification, the "halogen atom" includes fluorine, chlorine, bromine, and iodine.

In the present specification, the "polyether ring" refers to a non-aromatic hydrocarbon ring with two or more oxygen atoms that may have one or more substituents (e.g., -OH).

One or more pairs of two substituents in the substituted polyether ring may be linked to form alkanediyl (e.g., C2-C4 alkanediyl) that may have one or more substituents (e.g., -OH).

In the present specification, examples of the "non-aromatic hydrocarbon ring" include C3-C8 non-aromatic hydrocarbon rings (e.g., ethanediyl, propanediyl, and butanediyl). Specific examples include:
(1) C3-C8 cycloalkanes (e.g., cyclopropane, cyclobutane, cyclopentane, cyclohexane, cycloheptane, and cyclooctane);
(2) C5-C8 cycloalkenes (e.g., cyclopentene, cyclohexene, cycloheptene, and cyclooctene); and
(3) C5-C8 cycloalkadienes (e.g., cyclopentadiene, cyclohexadiene, cycloheptadiene, and cyclooctadiene).

In the present specification, examples of the "5- or 6-membered aromatic ring" include:
(1) a benzene ring that is a 6-membered aromatic hydrocarbon ring; and
(2) 5- or 6-membered monocyclic aromatic heterocyclic rings containing 1 to 3 heteroatoms selected from oxygen, sulfur, and nitrogen, in addition to carbon, as ring constituent atoms, such as a furan ring, thiophene ring, pyrrole ring, oxazole ring, isoxazole ring, thiazole ring, isothiazole ring, imidazole ring, pyrazole ring, 1,2,3-oxadiazole ring, 1,2,4-oxadiazole ring, 1,3,4-oxadiazole ring, furazane ring, 1,2,3-thiadiazole ring, 1,2,4-thiadiazole ring, 1,3,4-thiadiazole ring, 1,2,3-triazole ring, 1,2,4-triazole ring, tetrazole ring, pyridine ring, pyridazine ring, pyrimidine ring, pyrazine ring, and triazine ring.

In the present specification, unless otherwise specified, examples of the "alkyl group" include linear or branched C₁-C₁₀ alkyl groups. Specific examples include methyl, ethyl, propyl (e.g., n-propyl and isopropyl), butyl (e.g., n-butyl, isobutyl, sec-butyl, and tert-butyl), pentyl (e.g., n-pentyl, tert-pentyl, neopentyl, isopentyl, sec-pentyl, and 3-pentyl), hexyl, heptyl, octyl, nonyl, and decyl.

In the present specification, unless otherwise specified, the "alkoxy group" can be a group represented by RO-, wherein R is an alkyl group (preferably a C₁-C₃ alkyl group).

In the present specification, unless otherwise specified, the "alkylcarbonyloxy group" can be a group represented by RCO-O-, wherein R is an alkyl group (preferably a C₁-C₃ alkyl group) .

Specific examples include an acetoxy group.

In the present specification, unless otherwise specified, an example of the "alkanoyl group" is a group represented by RCO-, wherein R is an alkyl group (preferably a C₁-C₃ alkyl group).

Specific examples include an acetyl group.

In the present specification, the term "detection" can include measurement and quantification.

The present invention is described in detail below.

### 2. Acetylpolyamine detection agent

### 2.1. Means and mechanism of detection

The acetylpolyamine detection agent of the present invention comprises compound (A) having one or more polyether rings, wherein the polyether rings have one or more hydroxyl groups as substituents.

In the present invention, compounds (A) may be used singly or in combination of two or more.

Compound (A) can be a means for detecting acetylpolyamine.

The detection can be based on trapping of acetylpolyamine in the polyether ring of compound (A).

In the present specification, acetylpolyamine can be detected by detecting compound (A) that has trapped acetylpolyamine in the polyether ring.

For the detection, compound (A) may preferably have a coloring portion.

When at least one of the one or more hydroxyl groups contained in the polyether ring is a phenolic hydroxyl group, it is preferable that the coloring portion undergoes coloration based on the interaction between the phenolic hydroxyl group and the acetylpolyamine trapped in the polyether ring.

Preferable examples of such a compound having a coloring portion include a compound represented by formula (A0): 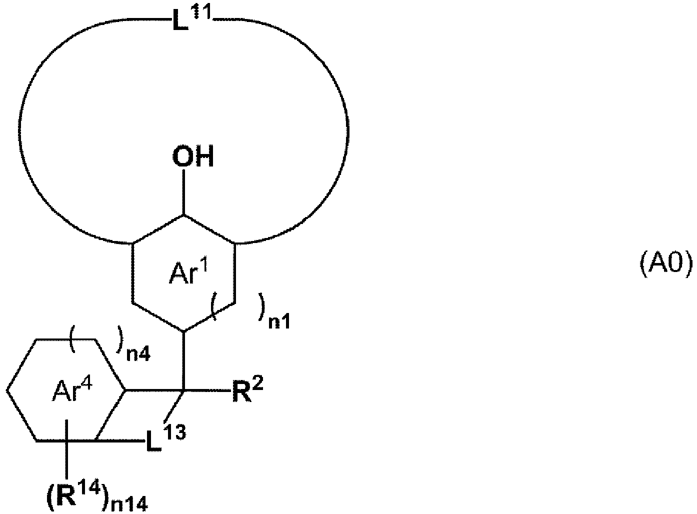 wherein
Ar¹ is a substituted or unsubstituted 5- or 6-membered aromatic ring,
n1 is 0 or 1,
Ar⁴ is a 5- or 6-membered aromatic ring,
n4 is 0 or 1,
R² is hydrogen, or a group represented by formula (A^{b2}): 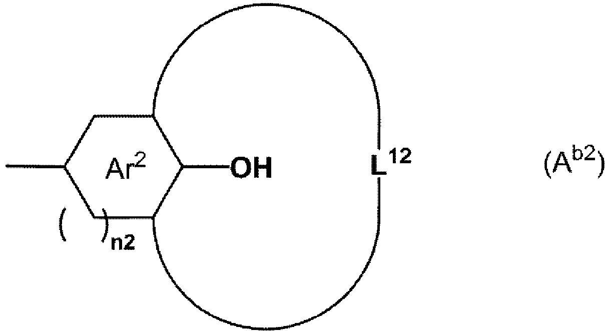 wherein
   Ar² is a substituted or unsubstituted 5- or 6-membered aromatic ring,
   n2 is 0 or 1, and
   L¹² is a divalent polyether chain;
L¹³ is a divalent group that can be cleaved at the end or inside, n¹⁴ is a number of 0 to 4, and
R¹⁴ is independently at each occurrence a substituent, or two of R¹⁴ may form a substituted or unsubstituted ring together with carbon atoms to which they are bonded
(in the present specification, this compound is also referred to as "compound (A0)").

In the present invention, compounds (A0) may be used singly or in combination of two or more.

For Ar¹, examples of substituents in the "substituted or unsubstituted 5- or 6-membered aromatic ring" include a hydroxy group, an alkoxy group (preferably a methoxy group), alkylcarbonyloxy (preferably an acetoxy group), an alkanoyl group (preferably an acetyl group), an alkyl group (preferably a methyl group), an amino group, a dialkylamino group (preferably a dimethylamino group), and a halo group (preferably a fluoro group, a chloro group, a bromo group, or an iodo group).

For Ar², examples of substituents in the "substituted or unsubstituted 5- or 6-membered aromatic ring" include a hydroxy group, an alkoxy group (preferably a methoxy group), alkylcarbonyloxy (preferably an acetoxy group), an alkanoyl group (preferably an acetyl group), an alkyl group (preferably a methyl group), an amino group, an alkylamino group (preferably a methylamino group), a dialkylamino group (preferably a dimethylamino group), a halo group (preferably a fluoro group, a chloro group, a bromo group, or an iodo group), and the like.

Ar¹ is preferably a benzene ring.

R² is preferably a group represented by the above formula (A^{b2}).

Ar² is preferably a benzene ring.

Compound (A) can be preferably a compound represented by formula (A1): 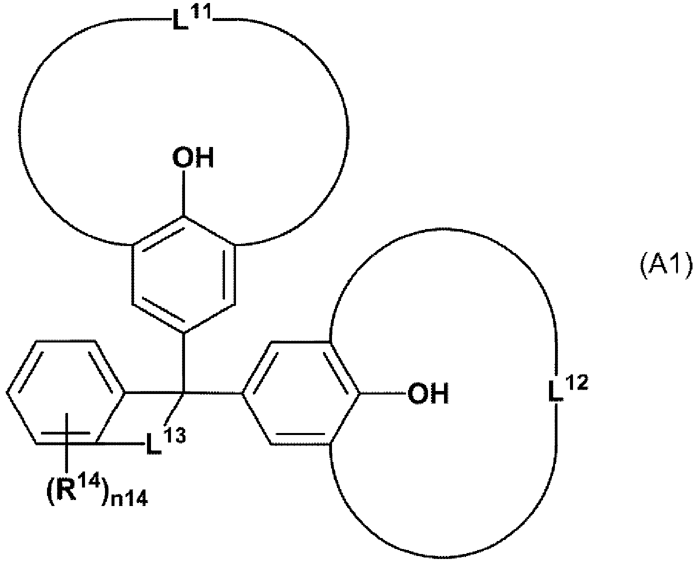 wherein
L¹¹ is a divalent polyether chain,
L¹² is a divalent polyether chain,
L¹³ is a divalent group that can be cleaved at the end or inside, n¹⁴ is a number of 0 to 4, and
R¹⁴ is independently at each occurrence a substituent, or two of R¹⁴ may form a substituted or unsubstituted ring together with carbon atoms to which they are bonded
(in the present specification, this compound is also referred to as "compound (A1)").

In the present invention, compounds (A1) may be used singly or in combination of two or more.

Each symbol in formula (A1) corresponds to the same symbol in formula (A0).

The compound represented by formula (A0) (and compound (A1) included therein) can form a colored complex in such a manner that L¹³ is cleaved based on the interaction between:
(a) the phenolic hydroxyl group shown in formula (A2), and
(b) the acetylpolyamine trapped in the polyether ring.

Specific examples thereof include an acid-base reaction between:
(a1) the phenolic hydroxyl group shown in formula (A2), and
(b1) the imino moiety of the acetylpolyamine.

L¹¹ can preferably have a chain length of 6 to 30, more preferably 9 to 27, and even more preferably 12 to 24 atoms.

L¹¹ can preferably have 2 to 10, more preferably 3 to 9, and even more preferably 4 to 8 oxygen atoms in the main chain.

When L¹¹ contains a cyclic structure, among chains with the largest number of constituent oxygen atoms, a single path with the smallest number of constituent atoms is regarded as the main chain.

L¹¹ can be preferably (1) or (2) :
(1) -CH₂-(OCH₂CH₂)ₙ₁₁₁-OCH₂-, wherein n111 is a number of 1 to 10 or 2 to 10, more preferably 3 to 9, and even more preferably 4 to 8; or
(2) -(Tz)ₙ₁₁₂-, wherein Tz is a glucose residue, and n112 is a number of 1 to 10 or 2 to 10, more preferably 3 to 9, and even more preferably 4 to 8.

L¹¹ can preferably have a chain length of 6 to 30, more preferably 9 to 27, and even more preferably 12 to 24 atoms.

L¹¹ can preferably have 2 to 10, more preferably 3 to 9, and even more preferably 4 to 8 oxygen atoms in the main chain.

When L¹¹ contains a cyclic structure, among chains with the largest number of constituent oxygen atoms, a single path with the smallest number of constituent atoms is regarded as the main chain.

L¹² can be preferably (1) or (2) :
(1) -CH₂-(OCH₂CH₂)ₙ₁₂₁-OCH₂-, wherein n121 is a number of 1 to 10, or
(2) -(Tz)ₙ₁₂₂-, wherein Tz is a glucose residue, and n122 is a number of 1 to 10.

L¹² can preferably have a chain length of 6 to 30, more preferably 9 to 27, and even more preferably 12 to 24 atoms.

L¹² can preferably have 2 to 10, more preferably 3 to 9, and even more preferably 4 to 8 oxygen atoms in the main chain.

When L¹² contains a cyclic structure, among chains with the largest number of constituent oxygen atoms, a single path with the smallest number of constituent atoms is regarded as the main chain.

L¹³ can preferably be -CO₂-, -SO₃-, or -CONH-. L¹³ can more preferably be -CO₂- or -SO₃-.

These preferable divalent groups are preferably arranged in a direction in which the -CO- or -SO₂- moiety is adjacent to the benzene ring shown in formula (A1).

0045 R¹⁴ is preferably, independently at each occurrence, a substituent selected from substituent group A, an alkyl group,
an aryl group (this group may be substituted with one or more substituents selected from substituent group A), or a heteroaryl group (this group may be substituted with one or more substituents selected from substituent group A), or two of them may be bonded to form an aromatic ring (this ring may be substituted with one or more substituents selected from substituent group A).

Substituent group A is a group consisting of:
a halogen atom,
a nitro group,
-CO₂R,
-CONH₂,
-CONHR,
-CONR₂
-SO₃H,
-SO₃R,
-SO₂NH₂,
-SO₂NHR,
-SO₂NR₂,
-NH₂,
-NHR,
-NR2,
-N⁺R₃
-OR,
-OH
-SR,
-SH, and
a linear or branched alkyl group, aryl group, or heteroaryl group each substituted with one or more halogen atoms (preferably a linear or branched alkyl group, and more preferably a linear or branched alkyl group having 1 to 4 carbon atoms).

In these formulas, R is independently at each occurrence a linear or branched alkyl group having 1 to 4 carbon atoms.

0046 R¹⁴ can be independently at each occurrence:
preferably a halogen atom, an alkyl group substituted with one or more fluorine atoms, NR₂, or -OR;
more preferably -F, -Cl, -Br, -CF₃, -NMe₂, or -OMe; and
even more preferably -NMe₂ or -OMe.

0047 R¹⁴ can be preferably an electron-withdrawing group.

0048 Particularly preferably, in formula (A1),
L¹¹ is a divalent polyether chain of 12 to 24 atoms (the chain contains 4 to 8 oxygen atoms);
L¹² is a divalent polyether chain of 12 to 24 atoms (the chain contains 4 to 8 oxygen atoms);
L¹³ is CO₂ or SO₃; and
R¹⁴ is -NMe₂ or -OMe.

0049

### 2.2. Method for producing compound (A0)

Compound (A0) is commercially available, or can be produced by the method for producing compound (A1) described below or by a similar method.

0050

### 2.2.1. Method for producing compound (A1)

Compound (A1) is commercially available, or can be produced by the method described below or by a similar method.

0051 Compound (A1) can be produced by the method described in the following scheme.

In the scheme, Y represents a protecting group (e.g., an allyl group) for the -OH group.

The method comprises:
step (a): reacting a compound of formula (1) with a compound of formula (2), which is lithiated with a lithiating agent (e.g., t-BuLi), to obtain a compound of formula (2) (in the present specification, this compound is also referred to as "compound (2)"); and
step (b): deprotecting compound (2) to obtain compound (A1) .

The reaction conditions for each step can be suitably set based on common technical knowledge about these methods.

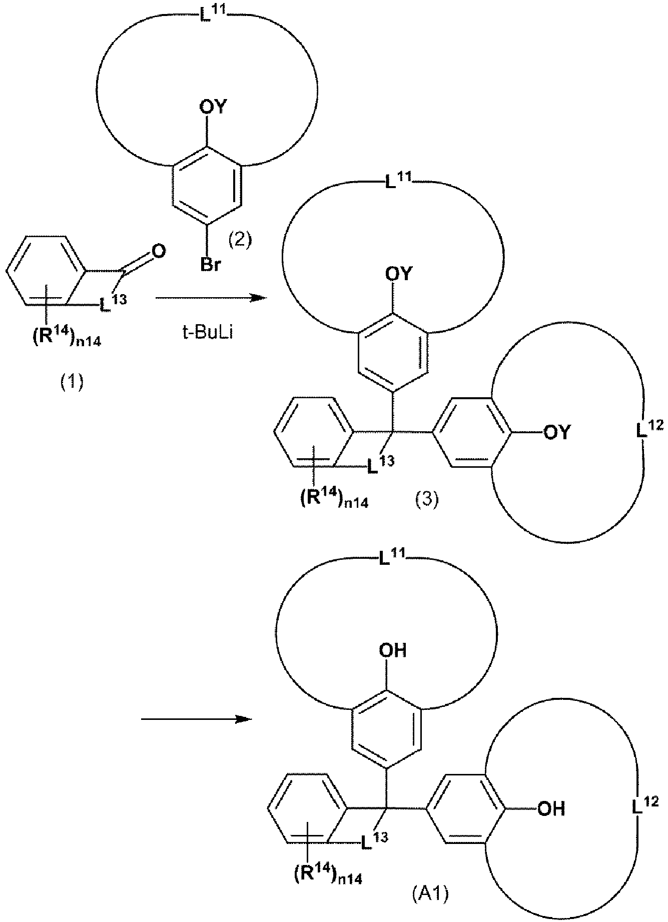

0052

### 2.3. Target and purpose of detection

The detection agent can be preferably used for detection of acetylpolyamine in a water-containing sample.

0053 The water-containing sample can be preferably a biological sample.

0054 Examples of the biological sample include biological samples derived from humans, monkeys, goats, sheep, rabbits, mice, rats, guinea pigs, or other mammals. Preferable examples include biological samples derived from humans.

Examples of the biological sample include body fluids (e.g., blood (e.g., serum and plasma), sweat, and urine), tissue extracts, and tissue-derived cells.

Among these, urine is preferred as the biological sample because acetylpolyamine is contained in urine and urine collection is less burdensome for the test subject.

The biological sample may be suitably subjected to filtration, dilution, or a combination thereof.

That is, the biological sample can be a sample derived from the body fluids described above.

0055 The detection agent of the present invention can be used for diagnosis of a disease associated with a change in the acetylpolyamine concentration of the body fluid of an affected patient.

The detection agent of the present invention can be a detection agent for detecting an acetylpolyamine concentration *in vitro* for diagnosis of a disease associated with a change in the acetylpolyamine concentration of the body fluid of an affected patient.

0056 In the present specification, the phrase "diagnosis of a disease" includes determination of the presence or degree of a disease, disorder, or symptom, or determination of risk of developing the disease, disorder, or symptom.

0057 Regarding the present invention, the diagnosis of a disease can be performed, for example, by comparing the acetylpolyamine concentration (T) of a body fluid obtained using a sample from a subject, with the acetylpolyamine concentration (H) in a healthy subject.

0058 When urine is used as a sample, the acetylpolyamine concentration can be corrected by the urinary creatinine concentration, if necessary.

0059 The acetylpolyamine concentration (H) in the healthy subject can be the average of the acetylpolyamine concentrations of body fluids obtained using samples from a statistically appropriate number of healthy subjects.

0060 Detection and quantification of acetylpolyamine can be carried out by visually observing the degree of coloration of reddish or bluish color, or by measuring absorption at one or more wavelengths in the range of 490 to 550 nm and 560 to 650 nm.

Detection and quantification of acetylpolyamine can also be carried out by detecting and quantifying a complex in which the polyamine is held, using devices such as NMR and IR.

The quantification can be performed, for example, by comparison with a standard or control whose acetylpolyamine concentration is known.

0061 For example, when concentration (T)/concentration (H) is 2.1-fold, 2.2-fold, 2.3-fold, 2.5-fold, 2.7-fold, or 3-fold or more, it can be used to determine, or can be used as a factor in determining, that the subject is suffering from the disease, has the possibility of suffering from the disease, or has the possibility of developing the symptoms of the disease.

0062 In addition, for example, when concentration (T) ≥ concentration (H) + (standard deviation of concentration (H)) x n, it can be used to determine, or can be used as a factor in determining, that the subject is suffering from the disease, has the possibility of suffering from the disease, or has the possibility of developing the symptoms of the disease.

In this case, n can be, for example, 1.5, 2, or 2.5.

0063 Specifically, for example, when concentration (T) ≥ 0.25 µmol/g-creatinine as the creatinine-corrected value, it can be used to determine, or can be used as a factor in determining, that there is the possibility that the subject has been suffering from a certain disease (e.g., cancer).

0064 Examples of the disease include cancer.

0065 In the present specification, tumor, cancer (carcinoma), and malignant neoplasm can be used interchangeably depending on the context.

0066 Examples of the disease include cancer (e.g., lung cancer, stomach cancer, colon cancer, liver cancer, pancreatic cancer, breast cancer, and prostate cancer) and tumor (e.g., malignant tumor).

0067 In another respect, examples of the disease include the following neoplasms (in particular, malignant neoplasms).
(1) Malignant neoplasm of lip, oral cavity and pharynx e.g., malignant neoplasm of lip, malignant neoplasm of base of tongue, malignant neoplasm of other and unspecified parts of tongue, malignant neoplasm of gum, malignant neoplasm of floor of mouth, malignant neoplasm of palate, malignant neoplasm of other and unspecified parts of mouth, malignant neoplasm of parotid gland, malignant neoplasm of other and unspecified major salivary glands, malignant neoplasm of tonsil, malignant neoplasm of oropharynx, malignant neoplasm of nasopharynx, malignant neoplasm of piriform sinus, malignant neoplasm of hypopharynx, malignant neoplasm of other and ill-defined sites in the lip, oral cavity and pharynx
(2) Malignant neoplasms of digestive organs e.g., malignant neoplasm of oesophagus, malignant neoplasm of stomach, malignant neoplasm of small intestine, malignant neoplasm of colon, malignant neoplasm of rectosigmoid junction, malignant neoplasm of rectum, malignant neoplasm of anus and anal canal, malignant neoplasm of liver and intrahepatic bile ducts, malignant neoplasm of gallbladder, malignant neoplasm of other and unspecified parts of biliary tract, malignant neoplasm of pancreas, malignant neoplasm of other and ill-defined digestive organs
(3) Malignant neoplasms of respiratory and intrathoracic organs e.g., malignant neoplasm of nasal cavity and middle ear, malignant neoplasm of accessory sinuses, malignant neoplasm of larynx, malignant neoplasm of trachea, malignant neoplasm of bronchus and lung, malignant neoplasm of thymus, malignant neoplasm of heart, mediastinum and pleura, malignant neoplasm of other and ill-defined sites in the respiratory system and intrathoracic organs
(4) Malignant neoplasms of bone and articular cartilage e.g., malignant neoplasm of bone and articular cartilage of limbs, malignant neoplasm of bone and articular cartilage of other and unspecified sites
(5) Melanoma and other malignant neoplasms of skin e.g., malignant melanoma of skin, other malignant neoplasms of skin
(6) Malignant neoplasms of mesothelial and soft tissue e.g., mesothelioma, Kaposi's sarcoma, malignant neoplasm of peripheral nerves and autonomic nervous system, malignant neoplasm of retroperitoneum and peritoneum, malignant neoplasm of other connective and soft tissue
(7) Malignant neoplasm of breast e.g., malignant neoplasm of breast
(8) Malignant neoplasms of female genital organs e.g., malignant neoplasm of vulva, malignant neoplasm of vagina, malignant neoplasm of cervix uteri, malignant neoplasm of corpus uteri, malignant neoplasm of uterus, part unspecified, malignant neoplasm of ovary, malignant neoplasm of other and unspecified female genital organs, malignant neoplasm of placenta
(9) Malignant neoplasms of male genital organs e.g., malignant neoplasm of penis, malignant neoplasm of prostate, malignant neoplasm of testis, malignant neoplasm of other and unspecified male genital organs
(10) Malignant neoplasms of urinary tract e.g., malignant neoplasm of kidney, except renal pelvis, malignant neoplasm of renal pelvis, malignant neoplasm of ureter, malignant neoplasm of bladder, malignant neoplasm of other and unspecified urinary organs
(11) Malignant neoplasms of eye, brain and other parts of central nervous system e.g., malignant neoplasm of eye and adnexa, malignant neoplasm of meninges, malignant neoplasm of brain, malignant neoplasm of spinal cord, cranial nerves and other parts of central nervous system
(12) Malignant neoplasms of thyroid and other endocrine glands e.g., malignant neoplasm of thyroid gland, malignant neoplasm of adrenal gland, malignant neoplasm of other endocrine glands and related structures
(13) Malignant neoplasms of ill-defined, secondary and unspecified sites e.g., malignant neoplasm of other and ill-defined sites, secondary and unspecified malignant neoplasm of lymph nodes, secondary malignant neoplasm of respiratory and digestive organs, secondary malignant neoplasm of other sites, malignant neoplasm without specification of site
(14) Malignant neoplasms of lymphoid, haematopoietic and related tissue e.g., Hodgkin's disease, follicular nodular non-Hodgkin's lymphoma, diffuse non-Hodgkin's lymphoma, peripheral and cutaneous T-cell lymphomas, other and unspecified types of non-Hodgkin's lymphoma, malignant immunoproliferative diseases, multiple myeloma and malignant plasma cell neoplasms, lymphoid leukaemia, myeloid leukaemia, monocytic leukaemia, other leukaemias of specified cell type, leukaemia of unspecified cell type, other and unspecified malignant neoplasms of lymphoid, haematopoietic and related tissue
(15) Malignant neoplasms of independent primary multiple sites e.g., malignant neoplasms of independent (primary) multiple sites
(16) In situ neoplasms e.g., carcinoma in situ of oral cavity, oesophagus and stomach, carcinoma in situ of other and unspecified digestive organs, carcinoma in situ of middle ear and respiratory system, melanoma in situ, carcinoma in situ of skin, carcinoma in situ of breast, carcinoma in situ of cervix uteri, carcinoma in situ of other and unspecified genital organs, carcinoma in situ of other and unspecified sites
(17) Benign neoplasms e.g., benign neoplasm of mouth and pharynx, benign neoplasm of major salivary glands, benign neoplasm of colon, rectum, anus and anal canal, benign neoplasm of other and ill-defined parts of digestive system, benign neoplasm of middle ear and respiratory system, benign neoplasm of other and unspecified intrathoracic organs, benign neoplasm of bone and articular cartilage, benign lipomatous neoplasm, haemangioma and lymphangioma, any site, benign neoplasm of mesothelial tissue, benign neoplasm of soft tissue of retroperitoneum and peritoneum, other benign neoplasms of connective and other soft tissue, melanocytic naevi, other benign neoplasms of skin, benign neoplasm of breast, leiomyoma of uterus, other benign neoplasms of uterus, benign neoplasm of ovary, benign neoplasm of other and unspecified female genital organs, benign neoplasm of male genital organs, benign neoplasm of urinary organs, benign neoplasm of eye and adnexa, benign neoplasm of meninges, benign neoplasm of brain and other parts of central nervous system, benign neoplasm of thyroid gland, benign neoplasm of other and unspecified endocrine glands, benign neoplasm of other and unspecified sites
(18) Neoplasms of uncertain or unknown behaviour e.g., neoplasm of uncertain or unknown behaviour of oral cavity and digestive organs, neoplasm of uncertain or unknown behaviour of middle ear and respiratory and intrathoracic organs, neoplasm of uncertain or unknown behaviour of female genital organs, neoplasm of uncertain or unknown behaviour of male genital organs, neoplasm of uncertain or unknown behaviour of urinary organs, neoplasm of uncertain or unknown behaviour of meninges, neoplasm of uncertain or unknown behaviour of brain and central nervous system, neoplasm of uncertain or unknown behaviour of endocrine glands, polycythaemia vera, myelodysplastic syndromes, other neoplasms of uncertain or unknown behaviour of lymphoid, haematopoietic and related tissue, neoplasm of uncertain or unknown behaviour of other and unspecified sites

0068 The detection agent can be preferably used for tumor diagnosis.

0069 Examples of the "acetylpolyamine" to be detected include monoacetylpolyamine and diacetylpolyamine.

0070 Specific examples of acetylpolyamine include N¹-acetylspermidine, N⁸-acetylspermidine, N¹,N⁸-diacetylspermidine, acetylspermine, and N¹,N¹²-diacetylspermine.

0071 Preferable examples of acetylpolyamine include diacetylpolyamine.

0072 Particularly preferable examples of acetylpolyamine include N¹,N¹²-diacetylspermine.

0073

### 3. Detection kit

The present invention also provides a detection kit comprising the detection agent of the present invention.

The kit may contain, in addition to compound (A1), at least one or more members selected from the group consisting of instructions, solvents (e.g., water and buffers), and diacetylpolyamine (which can be a control or standard).

0074 The detection kit of the present invention can be understood from the explanation about the acetylpolyamine detection agent of the present invention, other descriptions of the present specification, and common technical knowledge.

0075

### 4. Detection method

The present invention also provides a method for detecting acetylpolyamine, comprising bringing the detection agent of the present invention into contact with a sample having a possibility of containing acetylpolyamine.

0076 The contact may be achieved, for example, in the following manner:
(1) adding the detection agent of the present invention to a sample having a possibility of containing acetylpolyamine;
(2) adding a sample having a possibility of containing acetylpolyamine to the detection agent of the present invention; or
(3) mixing a sample having a possibility of containing acetylpolyamine and the detection agent of the present invention.

The detection method of the present invention can be understood from the explanation about the acetylpolyamine detection agent of the present invention, other descriptions of the present specification, and common technical knowledge.

0077

### 5. Composition

The present invention also provides a composition comprising compound (A1) or a ring-opened form thereof.

In addition to compound (A1), the composition may contain, for example, a solvent (e.g., water or a buffer).

The composition of the present invention can be understood from the explanation about the acetylpolyamine detection agent of the present invention, other descriptions of the present specification, and common technical knowledge.

0078

### 6. Complex

0079 The present invention also provides a complex comprising:
a compound represented by formula (A1) or a ring-opened form thereof: 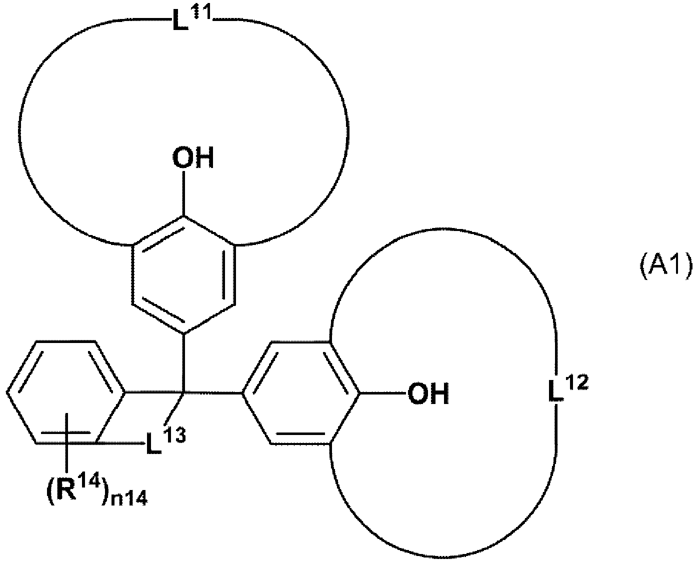 wherein
L¹¹ is a divalent polyether chain,
L¹² is a divalent polyether chain,
L¹³ is a divalent group that can be cleaved at the end or inside, n¹⁴ is a number of 0 to 4, and
R¹⁴ is independently at each occurrence a substituent, or two of R¹⁴ may form a substituted or unsubstituted ring together with carbon atoms to which they are bonded; and
   acetylpolyamine;
wherein the acetylpolyamine is held in at least one of the ring containing L¹¹ and the ring having L¹² of the compound represented by formula (A1) or the ring-opened form thereof.

0080 The complex can be understood from the explanation about the acetylpolyamine detection agent of the present invention, other descriptions of the present specification, and common technical knowledge.

The complex can be produced by bringing acetylpolyamine into contact with the compound represented by formula (A1) so that compound (A) is trapped in the polyether ring.

The method can be understood from the explanation about the acetylpolyamine detection agent of the present invention, other descriptions of the present specification, and common technical knowledge.

### Examples

0081 The present invention is described in more detail below with reference to Examples; however, the present invention is not limited thereto.

0082 The meanings of the symbols and abbreviations in the Examples are shown below.
Detection reagent 1: a compound having the structure of the following formula:

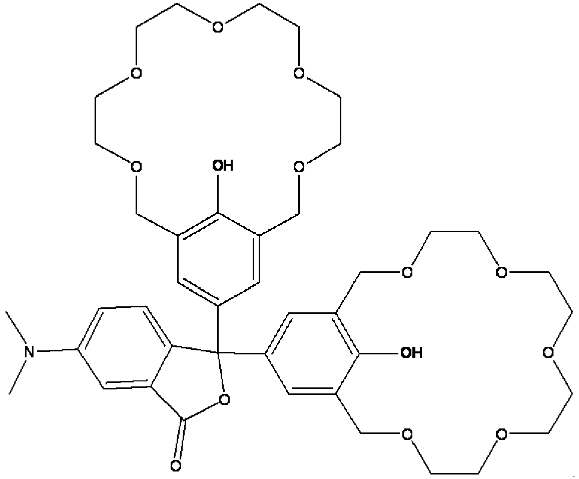

0083

### Example 1

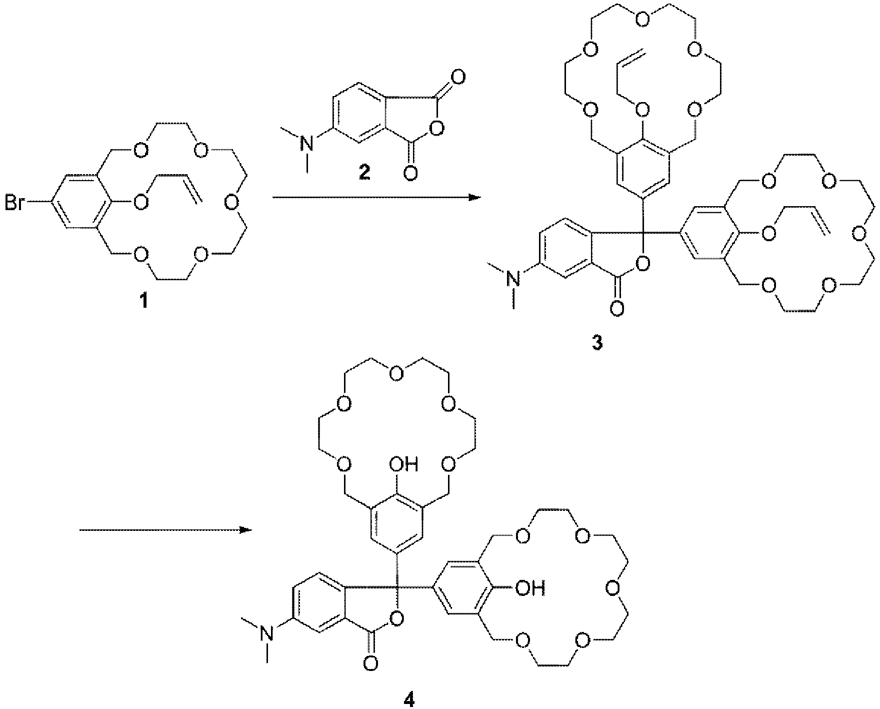

Detection reagent 1 can be obtained, for example, in the following manner. Polyether 1 was dissolved in 600 mg of tetrahydrofuran, and tert-butyllithium (n-pentane solution) was added under cooling. After stirring under cooling, a solution of dimethylaminophthalic acid 2 (130 mg) in tetrahydrofuran was added, and the mixture was stirred at room temperature for several hours. Then, 1N hydrochloric acid was added to stop the reaction, followed by ordinary extraction and purification by column chromatography, thereby obtaining 200 mg of compound 3. Then, compound 3 was redissolved in methanol, and 0.02 equivalents of Pd(PPh3)4 and 1.5 equivalents of sodium borohydride were added and stirred. Thereafter, the reaction was stopped with a 1N aqueous hydrochloric acid solution, followed by ordinary extraction and purification by column chromatography, thereby obtaining 150 mg of compound 4 (detection reagent 1).

The coloration of N¹,N¹²-diacetylspermine, which was the detection target of detection reagent 1, was examined at the various concentrations shown in Table 1. Table 1 shows the results.

1 mg of reagent 1 was weighed, dissolved in 3.1 mL, and diluted to 400 µM to obtain solution A. Solution A was diluted 1000-fold to obtain solution B.

2.5 mg of N¹,N¹²-diacetylspermine dihydrochloride was dissolved in 5 mL of methanol, and 1 mL of the resulting solution was dissolved in 2.4 mL of methanol to prepare 400 µL of solution, followed by neutralization with 2 equivalents of an aqueous NaOH solution to obtain solution C. Solution C was diluted 1000-fold to obtain solution D.

In Run 1, 1 mL of solution A and 1 mL of solution C were mixed. In Run 2, 1 mL of solution B and 1 mL of solution D were mixed. In Run 3, 1 mL of solution A and 1 mL of solution D were mixed.

The coloration of reddish color was confirmed by visual observation with the following criteria.

++: Transparent pink coloration was observed.

+: Transparent pale pink coloration was observed.

-: No coloration was observed (colorless and transparent)

A low concentration (200 nM) of detection target could be detected with 200 µM of detection reagent 1 (Run 3). In comparison, in the test for a high concentration (200 µM) of detection target, a coloration of darker color was observed (Run 1). Therefore, it was also confirmed that the density of the detection target could be detected by the intensity of coloration.

0084

**Table 1**

| Run | Detection reagent 1 | N¹,N¹²-diacetylspermine | Coloration |
|---|---|---|---|
| 1 | 200 µM | 200 µM | ++ |
| 2 | 200 nM | 200 nM | - |
| 3 | 200 µM | 200 nM | + |

## Claims

1. An acetylpolyamine detection agent comprising compound (A) having one or more polyether rings, wherein the polyether rings have one or more hydroxyl groups as substituents.

2. The acetylpolyamine detection agent according to claim 1, wherein compound (A) is a compound represented by formula (A1): 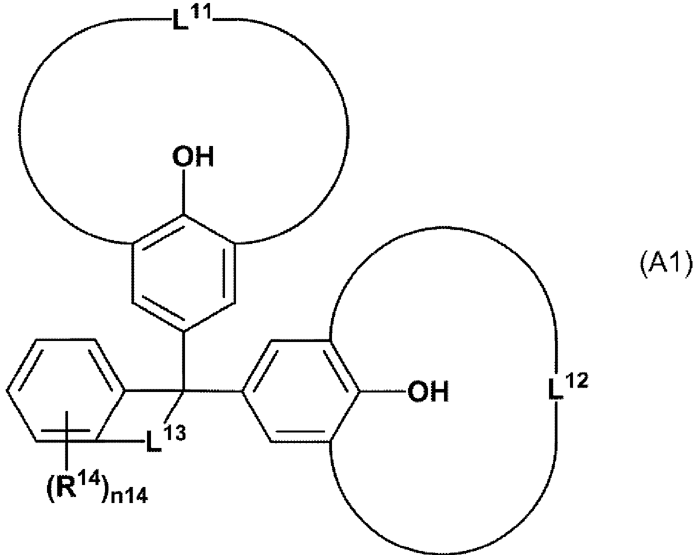 wherein
L¹¹ is a divalent polyether chain,
L¹² is a divalent polyether chain,
L¹³ is a divalent group that can be cleaved at the end or inside,
n¹⁴ is a number of 0 to 4, and
R¹⁴ is independently at each occurrence a substituent, or two of R¹⁴ may form a substituted or unsubstituted ring together with carbon atoms to which they are bonded.

3. The detection agent according to claim 2, wherein L¹¹ is:
(1) -CH₂-(OCH₂CH₂)ₙ₁₁₁-OCH₂-, wherein n111 is a number of 1 to 10, or
(2) -(Tz)ₙ₁₁₂-, wherein Tz is a glucose residue, and n112 is a number of 1 to 10.

4. The detection agent according to claim 2 or 3, wherein L¹² is:
(1) -CH₂-(OCH₂CH₂)ₙ₁₂₁-OCH₂-, wherein n121 is a number of 1 to 10, or
(2) -(Tz)ₙ₁₂₂-, wherein Tz is a glucose residue, and n122 is a number of 1 to 10.

5. The detection agent according to any one of claims 2 to 4, wherein L¹³ is -CO₂-, -SO₃-, or -CONH-.

6. The detection agent according to any one of claims 2 to 5, wherein R¹⁴ is independently at each occurrence -F, -Cl, -Br, - CF₃, -NMe₂, or -OMe.

7. The detection agent according to any one of claims 1 to 6, for use in detection of acetylpolyamine in a water-containing sample.

8. The detection agent according to any one of claims 1 to 7, wherein the water-containing sample is a biological sample.

9. The detection agent according to any one of claims 1 to 8, for use in diagnosis of a disease associated with a change in the acetylpolyamine concentration of the body fluid of an affected patient.

10. The detection agent according to any one of claims 1 to 9, for use in tumor diagnosis.

11. The detection agent according to any one of claims 1 to 10, wherein the acetylpolyamine is diacetylpolyamine.

12. The detection agent according to any one of claims 1 to 11, wherein the acetylpolyamine is N¹,N¹²-diacetylspermine.

13. A detection kit comprising the detection agent according to any one of claims 1 to 12.

14. A method for detecting acetylpolyamine, comprising bringing the detection agent according to any one of claims 1 to 12 into contact with a sample having a possibility of containing acetylpolyamine.

15. A composition comprising:
a compound represented by formula (A1) or a ring-opened form thereof: 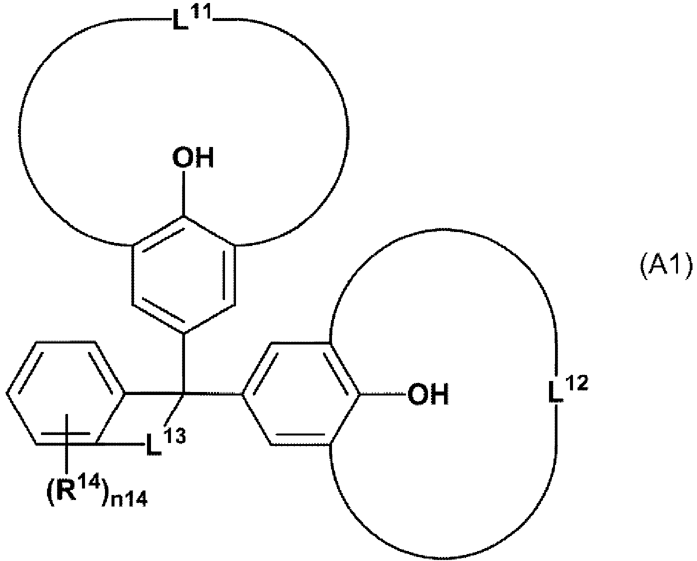 wherein
L¹¹ is a divalent polyether chain,
L¹² is a divalent polyether chain,
L¹³ is a divalent group that can be cleaved at the end or inside, n¹⁴ is a number of 0 to 4, and
R¹⁴ is independently at each occurrence a substituent, or two of R¹⁴ may form a substituted or unsubstituted ring together with carbon atoms to which they are bonded; and
acetylpolyamine.

16. A complex comprising:
a compound represented by formula (A1) or a ring-opened form thereof: 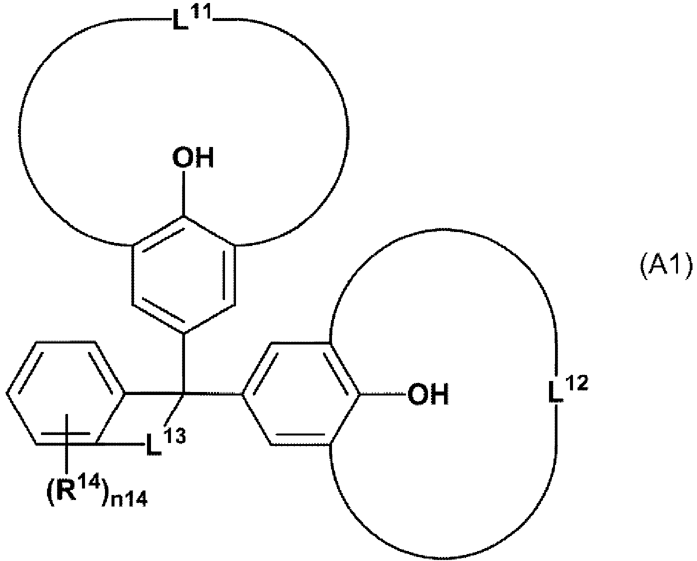 wherein
L¹¹ is a divalent polyether chain,
L¹² is a divalent polyether chain,
L¹³ is a divalent group that can be cleaved at the end or inside, n¹⁴ is a number of 0 to 4, and
R¹⁴ is independently at each occurrence a substituent, or two of R¹⁴ may form a substituted or unsubstituted ring together with carbon atoms to which they are bonded; and
acetylpolyamine;
wherein the acetylpolyamine is held in at least one of the ring containing L¹¹ and the ring having L¹² of the compound represented by formula (A1) or the ring-opened form thereof.
